(19) Europäisches Patentamt
European Patent Office
Office européen des brevets

(11) Veröffentlichungsnummer: **0 005 783**
**B1**

(12) **EUROPÄISCHE PATENTSCHRIFT**

(45) Veröffentlichungstag der Patentschrift: **30.12.81**

(21) Anmeldenummer: **79101541.5**

(22) Anmeldetag: **21.05.79**

(51) Int. Cl.³: **C 07 D 513/04,** A 61 K 31/415, C 07 D 417/04 // (C07D513/04, 285/00, 235/00)

# (54) Neue Imidazo(2.1-b)-(1.3.4)-thiadiazole, Verfahren zu ihrer Herstellung und diese enthaltende Arzneimittel.

(30) Priorität: **31.05.78 DE 2823686**

(43) Veröffentlichungstag der Anmeldung: **12.12.79 Patentblatt 79/25**

(45) Bekanntmachung des Hinweises auf die Patenterteilung: **30.12.81 Patentblatt 81/52**

(84) Benannte Vertragsstaaten: **AT BE CH DE FR GB IT NL SE**

(56) Entgegenhaltungen:
**CHEMICAL ABSTRACTS, vol. 47, Nr. 13, 10-07-1953, Zusammenfassung 6409g. Columbus, Ohio, USA T. MATSUKAWA "Chemotherapeutics XXVII. Reaction of a-bromoacetophenone with 2-amino-1,3,4-thiadiazoles".**

**CHEMICAL ABSTRACTS, vol. 68, Nr. 1, 01-01-1968, Zusammenfassung 2856m. Columbus, Ohio, USA N. SALDABOLS: "Synthesis in the methyl 2-furyl ketone series. XII. 5-nitro-2-furyl substituted imidazoheterocyclic compounds with a common nitrogen atom", Seite 269, Spalte 2.**

(73) Patentinhaber: **BAYER AG, Zentralbereich Patente, Marken und Lizenzen, D-5090 Leverkusen 1, Bayerwerk (DE)**

(72) Erfinder: **Horstmann, Harald, Dr., Claudiusweg 19, D-5600 Wuppertal 1 (DE)**
Erfinder: **Meng, Karl-August, Dr., Pahlkestrasse 15, D-5600 Wuppertal 1 (DE)**
Erfinder: **Seuter, Friedel, Dr., Moospfad 16, D-5600 Wuppertal 1 (DE)**
Erfinder: **Möller, Eike, Dr., Pahlkestrasse 37, D-5600 Wuppertal 1 (DE)**

(56) Entgegenhaltungen:
**JOURNAL OF HETEROCYCLIC CHEMISTRY, Band 2; September 1965, Albuquerque, New Mexico, USA L.M. WERBEL: "Synthesis of fused imidazo-heterocyclic systems", Seiten 287 bis 290.**

**EP 0 005 783 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

Neue Imidazo [2.1-b]-[1.3.4]-thiadiazole, Verfahren zu ihrer Herstellung und diese enthaltende Arzneimittel

Die vorliegende Erfindung betrifft neue Imidazo [2.1-b]-[1.3.4]-thiadiazole, Verfahren zu ihrer Herstellung sowie ihre Verwendung in Arzneimitteln, insbesondere in Antithrombotika und Thrombolytika.

Es ist bereits bekanntgeworden, dass einige Imidazo[2.1-b]-[1.3.4]-thiadiazolderivate chemotherapeutische, insbesondere antimikrobielle Eigenschaften besitzen.

(Vgl. Matsukawa und Ban, J. Pharm. Soc. Japan, 72, 610 (1952); C.A. 47, 6409 (1953).

Matsukawa et al., J. Pharm. Soc. Japan 73, 159 (1953); C.A. 47, 11185 (1950).

Ban, J. Pharm. Soc. Japan 74, 658 (1954); C.A. 48, 10740 (1954).

Ban, J. Pharm. Soc. Japan 74, 1044 (1954); C.A. 49, 11630 (1955).

Ihre Verwendung in Antithrombotika oder Antiphlogistika ist jedoch neu und bisher nicht bekanntgeworden.

Gegenstand der vorliegenden Anmeldung sind neue Imidazo-[2.1-b]-[1.3.4]-thiadiazole der allgemeinen Formel I

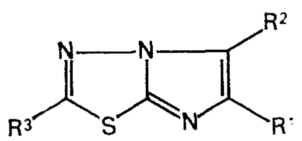

(I)

in welcher

$R^1$ für einen Phenylrest steht, der gegebenenfalls substituiert ist durch ein oder zwei gleiche oder verschiedene Substituenten aus der Gruppe Halogen, Trifluormethyl, Alkyl mit 1-6 C-Atomen, Alkoxy oder $SO_n$-Alkyl (n = 0 oder 2) mit jeweils 1-4 C-Atomen, Phenyl, Cyano, N-Methylsulfonamido, N-Dimethylsulfonamido oder Sulfonamido, deren N-Atom Glied eines 5-7 gliedrigen Heterocyclus wie Pyrolidino, Piperidino oder Hexamthylenimino ist und der gegebenenfalls ein weiteres Sauerstoffatom enthalten kann.

$R^2$ für Wasserstoff, einen Alkylrest mit 1-3 C-Atomen oder einen Phenylrest, der unsubstituiert oder durch Fluor, Chlor, Methyl, Äthyl, Methoxy oder Äthoxy substituiert sein kann, steht,

und

$R^3$ für α- oder β-Naphthyl, einen α- oder β-Furylrest, der durch ein oder zwei Methyl- oder Äthylreste substituiert sein kann, einen α- oder β-Thienylrest, der durch ein oder zwei Methyl- oder Äthylreste substituiert sein kann, einen α- oder β-Pyridylrest, der durch Methyl oder Äthyl substituiert sein kann, einen Methylendioxybenzylrest oder für einen Substituenten der Formel

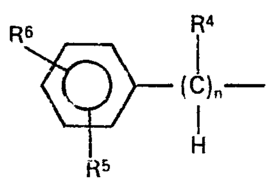

steht,

worin

n 0 oder 1 bedeutet,

$R^4$ Wasserstoff oder eine Methylgruppe bedeutet,

$R^5$ einen Substituenten aus der Gruppe Fluor, Chlor, Brom, Alkyl mit 1-4 C-Atomen oder Allyl, Phenyl, Trifluormethyl, Hydroxy, Alkoxy mit 1-4 C-Atomen, $SO_n$-Alkyl (n = 0-2) mit 1-2 C-Atomen im Alkylrest, Acyloxy mit bis zu 4 C-Atomen, Alkoxylencarboxy mit bis zu 6 C-Atomen, Oxyalkylencarbalkoxy mit bis zu 10 C-Atomen, Nitro, Cyano, Carboxy, Carbalkoxy mit bis zu 4 C-Atomen, bedeutet, oder für den Fall, dass n = 1 ist, Wasserstoff bedeutet,

$R^6$ Wasserstoff oder einen weiteren Substituenten aus der Gruppe Fluor, Chlor, Brom, Alkyl mit 1-4 C-Atomen oder einen Methoxy- oder Äthoxyrest bedeutet.

Es wurde gefunden, dass man Imidazo-[2.1-b]-[1.3.4]-thiadiazole der allgemeinen Formel I

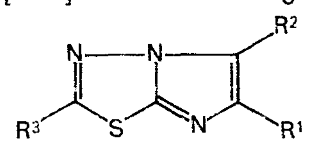

(I)

in welcher $R^1$, $R^2$ und $R^3$ die oben angegebene Bedeutung haben, erhält, wenn man

A) Carbonylverbindungen der allgemeinen Formel II

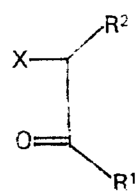

(II)

in welcher

$R^1$ und $R^2$ die oben angegebene Bedeutung haben und

X für eine austretende Gruppe, insbesondere für Halogen, Hydroxy, Alkoxy, Acyloxy, Alkyl, Arylsulfonyloxy, quartäres Ammonium, tertiäres Phosphonium oder Sulfonium steht,

wobei die Carbonylverbindungen der Formel II auch in Form ihrer entsprechenden Enamine, Enolester oder Enoläther vorliegen können,

mit 2-Amino-1.3.4-thiadiazolen der allgemeinen Formel III

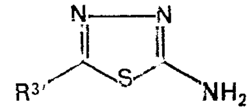

(III)

in welcher

$R^{3'}$ für Naphthyl, Furyl, Thienyl oder Pyridyl steht, die gegebenenfalls durch 1 oder 2 Methyl- oder Äthylreste substituiert sind, einen Methylendioxybenzylrest oder für einen Substituenten der allgemeinen Formel

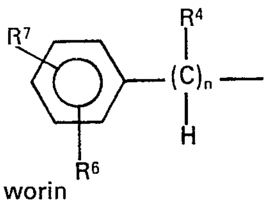

worin
n, $R^4$ und $R^6$ die oben angegebene Bedeutung haben und
$R^7$ für Fluor, Chlor, Brom, Alkyl, Allyl, Phenyl, Trifluormethyl, Hydroxy, Alkoxy, wobei die genannten Alkyl- und Alkoxyreste jeweils 1–4 C-Atome enthalten, $SO_n$-Alkyl (n = 0, 1 oder 2) mit 1 oder 2 C-Atomen im Alkylrest, Nitro oder Cyano steht, gegebenenfalls in Gegenwart inerter Lösungsmittel und basischer Hilfsstoffe bei Temperaturen zwischen 30 und 230°C umsetzt und gegebenenfalls in einer Nachfolgereaktion die Verbindungen der allgemeinen Formel I in welcher

a) $R^5$ für eine Hydroxylgruppe steht, diese Hydroxylgruppe in an sich bekannter Weise alkyliert oder acyliert wird oder

b) für den Fall, dass $R^5$ eine Nitrilgruppe bedeutet, diese Nitrilgruppe in an sich bekannter Weise zur Carboxygruppe verseift wird und gegebenenfalls anschliessend zu einer Carbalkoxygruppe verestert wird,

oder

B) wenn man 1-Acylamino-2-mercapto-imidazole der allgemeinen Formel IV

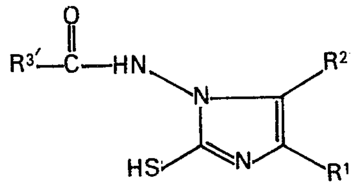

(IV)

in welcher
$R^1$, $R^2$ und $R^{3'}$ die oben angegebene Bedeutung haben,
mit wasserabspaltenden Mitteln, vorzugsweise mit anorganischen Säurehalogeniden, insbesondere mit Phosphorxychlorid, bei Temperaturen zwischen 30 und 200°C, gegebenenfalls unter erhöhtem Druck, cyclisiert.

Je nach Art der Methode und der verwendeten Ausgangsstoffe kann die Herstellung der erfindungsgemässen Verbindungen der Formel (I) durch folgendes Formelschema wiedergegeben werden, wobei 2-(4-Chlorphenyl)-6-phenylimidazo-[2.1-b]-[1.3.4]-thiadiazol als Beispiel gewählt wurde:

Formelschema:

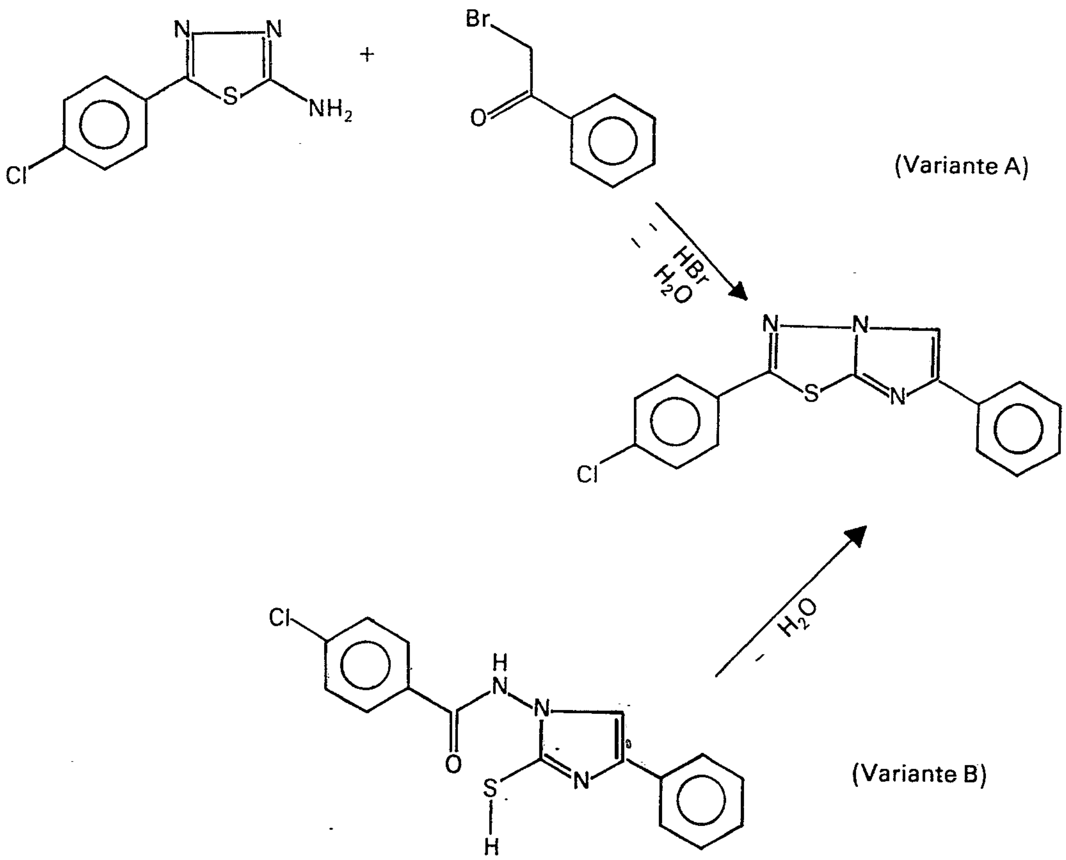

(Variante A)
(Variante B)

Bei der Umsetzung der Carbonylverbindung der allgemeinen Formel (II) mit 2-Amino-1.3.4-thiadiazol der allgemeinen Formel (III) entsteht intermediär die Verbindung der allgemeinen Formel (VI)

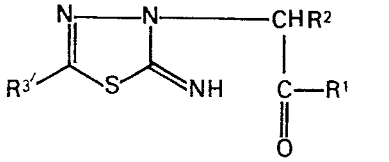

(IV)

wobei dieses Imid für den Fall, dass der austretende Rest X der Rest einer starken Säure ist, dass entsprechende Ammoniumsalz der Formel (V)

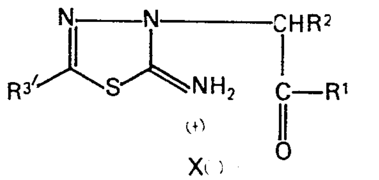

(V)

anfällt. Diese Zwischenstufen können gegebenenfalls isoliert werden und in einem zweiten Reaktionsschritt in üblicher Weise zu den erfindungsgemässen Verbindungen der Formel (I) cyclisiert werden. Von besonderem Interesse ist jedoch die Umsetzung von Verbindungen der Formel (II) mit Verbindungen der Formel (III) einstufig durchzuführen und die Cyclisierung ohne Isolierung der Zwischenstufen, insbesondere unter Verwendung hochsiedender Lösungsmittel, direkt durchzuführen.

Die austretende Gruppe X steht vorzugsweise für Halogen, Hydroxy, Alkoxy, Acyloxy, Alkyl, Arylsulfonyloxy, quartäres Ammonium, tertiäres Phosphonium oder Sulfonium.

Als basische Hilfsmittel bei der Umsetzung von II mit III werden vorzugsweise Alkali- oder Erdalkalihydroxyde wie Natriumhydroxyd, Bariumhydroxyd oder Calciumhydroxyd oder Alkali- und Erdalkalicarbonate wie Natriumcarbonat, Natriumhydrogencarbonat, Bariumcarbonat und Calciumcarbonat, oder organische Basen, wie Triäthylamin oder Pyridin eingesetzt.

Verbindungen der Formel (I) in denen $R^5$ eine Acyloxygruppe bedeutet, werden vorteilhafterweise dadurch hergestellt, dass man entsprechende Verbindungen der Formel (I) in welcher $R^5$ für Hydroxy steht mit Säureanhydriden oder Säurechloriden in an sich bekannter Weise acyliert.

Verbindungen in denen $R^5$ für einen Alkoxylencarboxy- oder Alkoxylencarbalkoxygruppe steht werden aus den entsprechenden Hydroxyverbindungen der Formel (I) durch Umsetzung mit α-Halogencarbonsäuren bzw. α-Halogencarbonsäureestern in an sich bekannter Weise gegebenenfalls in Gegenwart basischer Kondensationsmittel erhalten.

Verbindungen der allgemeinen Formel (I), in denen $R^5$ eine Carboxygruppe bedeutet, werden vorzugsweise hergestellt durch Verseifung der entsprechenden Nitrilverbindung oder durch Hydrolyse der entsprechenden Carbalkoxyverbindungen.

Andererseits lassen sich die entsprechenden Carbalkoxyverbindungen durch allgemein übliche bekannte Veresterungsverfahren aus den Verbindungen, in denen $R^5$ eine Carboxygruppe darstellt, durch Umsetzung mit entsprechenden Alkoholen gewinnen.

Falls nicht ausdrücklich anders angegeben, steht Halogen für Fluor, Chlor oder Brom.

Alkyl steht vorzugsweise für geradkettiges, verzweigtes oder cyclisches Alkyl mit bis zu 6 Kohlenstoffatomen, wobei die Alkylenkette gegebenenfalls durch Sauerstoff unterbrochen ist.

Alkenyl steht vorzugsweise für geradkettiges, verzweigtes oder cyclisches Alkenyl mit 1 oder 2 ungesättigten Bindungen und mit bis zu 6 Kohlenstoffatomen, wobei gegebenenfalls eine $CH_2$-Gruppe durch Sauerstoff ersetzt ist.

Der Acylrest in den beanspruchten Acyloxygruppen steht vorzugsweise für einen Alkanoylrest mit 1 bis 4 Kohlenstoffatomen oder für einen gegebenenfalls substituierten Benzoylrest.

Das Stickstoffatom der Sulfonamidogruppe ist vorzugsweise unsubstituiert oder durch 1 oder 2 Alkylreste mit 1 bis 4 Kohlenstoffatomen substituiert, wobei die beiden Alkylreste gegebenenfalls einen 5 bis 8gliedrigen Ring bilden, der 1 oder 2 gleiche oder verschiedene Heteroatome aus der Gruppe Sauerstoff, Schwefel oder N-Z enthält, wobei Z für eine Alkylgruppe mit 1 bis 4 Kohlenstoffatomen oder für einen gegebenenfalls substituierten Phenylrest steht.

Unter einer Alkoxylencarboxygruppe wird ein Substituent der Formel

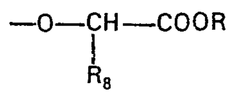

und unter einer Alkoxylencarbalkoxygruppe ein Substituent der Formel

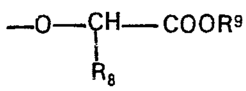

verstanden,
wobei
$R^8$ vorzugsweise Wasserstoff oder einen geradkettigen oder verzweigten Alkylrest mit 1 bis 4 C-Atomen bedeutet und
$R^9$ vorzugsweise einen geradkettigen oder verzweigten Alkylrest mit 1 bis 4 C-Atomen bedeutet.

Unter der Carbalkoxygruppe wird vorzugsweise ein Substituent der Formel

$-COOR^9$

verstanden,
wobei
$R^9$ die oben angegebene Bedeutung hat.

Unter Furyl, Thienyl, Pyridyl und Naphthyl werden vorzugsweise solche Reste verstanden, die in α- bzw. β-Position verknüpft sind.

Von besonderer Bedeutung sind erfindungsgemässe Verbindungen der allgemeinen Formel (I), worin

$R^1$ für Phenyl steht, das gegebenenfalls durch Fluor, Chlor, Methyl, tert.-Butyl, Phenyl, Trifluormethyl, Methoxy, Methylmercapto, Methylsulfonyl oder die

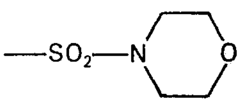 Gruppe substituiert ist,

$R^2$ für Wasserstoff, einen Methyl- oder Phenylrest steht,

$R^3$ α- oder β-Naphthyl, α- oder β-Furyl oder Thienyl bzw. α- oder β-Pyridyl bedeutet, wobei die heterocyclischen Reste durch eine Methylgruppe substituiert sein können, oder für einen Rest der Formel

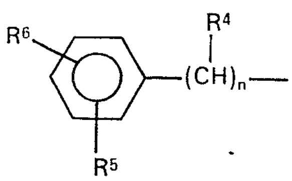

worin

n 0 oder 1 und

$R^4$ Wasserstoff oder eine Methylgruppe bedeutet und

$R^5$ für Methyl, Äthyl, Propyl, Isopropyl, Trifluormethyl, Fluor, Chlor oder Brom, Hydroxy, Methoxy, Äthoxy oder Propoxy, Oxymethylencarboxy, Oxymethylencarbomethoxy, Oxymethylencarbäthoxy, α-Oxyäthylencarboxy, α-Oxyäthylencarbomethoxy, α-Oxyäthylencarboäthoxy, Methylmercapto, Methylsulfonyl, Acetoxy, Propionyloxy, Nitro, Cyano, Carboxy, Carbomethoxy oder Carboäthoxy bedeutet und

$R^6$ Wasserstoff, Methyl oder Äthyl, Fluor, Chlor, Methoxy oder Äthoxy bedeutet.

In den Formeln (III) und (IV) steht $R^3$ vorzugsweise für Naphthyl, Furyl, Thienyl oder Pyridyl, jeweils in α- oder β-Stellung, wobei die heterocyclischen Ringe gegebenenfalls durch eine Methylgruppe substituiert sind,

oder für den Rest der Formel

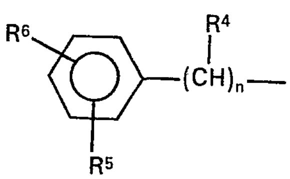

worin

n 0 oder 1 bedeutet,

$R^4$ Wasserstoff oder eine Methylgruppe bedeutet,

$R^5$ für Alkyl mit 1 bis 4 Kohlenstoffatomen, Trifluormethyl, Fluor, Chlor, Brom, Hydroxy, Alkoxy mit 1 bis 4 Kohlenstoffatomen, Methylmercapto, Methylsulfonyl, Nitro oder Cyano steht und

$R^6$ für Wasserstoff, Alkyl oder Alkoxy mit je 1 bis 2 Kohlenstoffatomen, Fluor oder Chlor steht.

Die als Ausgangsprodukte verwendeten Thiadiazole der Formel (III) sind bekannt oder können nach bekannten Methoden hergestellt werden (Lit.: Freund u. Meinecke, Ber. 29, 2511 (1896); Young u. Eyre, J. Chem. Soc. 79, 54 (1901)

2-Amino-5-[3-methyl-furyl-(2)]-1.3.4-thiadiazol
2-Amino-5-[4-methyl-furyl-(2)]-1.3.4-thiadiazol
2-Amino-5-[5-methyl-furyl-(2)]-1.3.4-thiadiazol
2-Amino-5-[2-methyl-furyl-(3)]-1.3.4-thiadiazol
2-Amino-5-[4-methyl-furyl-(3)]-1.3.4-thiadiazol
2-Amino-5-[5-methyl-furyl-(3)]-1.3.4-thiadiazol
2-Amino-5-[3,4-dimethyl-furyl-(2)]-1.3.4-thiadiazol
2-Amino-5-[3,5-dimethyl-furyl-(2)]-1.3.4-thiadiazol
2-Amino-5-[3-methyl-thienyl-(2)]-1.3.4-thiadiazol
2-Amino-5-[4-methyl-thienyl-(2)]-1.3.4-thiadiazol
2-Amino-5-[5-methyl-thienyl-(2)]-1.3.4-thiadiazol
2-Amino-5-[2-methyl-thienyl-(3)]-1.3.4-thiadiazol
2-Amino-5-[4-methyl-thienyl-(3)]-1.3.4-thiadiazol
2-Amino-5-[5-methyl-thienyl-(3)]-1.3.4-thiadiazol
2-Amino-5-[3.4-dimethyl-thienyl-(2)]-1.3.4-thiadiazol
2-Amino-5-[3.5-dimethyl-thienyl-(2)]-1.3.4-thiadiazol
2-Amino-5-[2.5-dimethyl-thienyl-(3)]-1.3.4-thiadiazol
2-Amino-5-[4.5-dimethyl-thienyl-(2)]-1.3.4-thiadiazol
2-Amino-5-[4.5-dimethyl-furyl-(2)]-1.3.4-thiadiazol
2-Amino-5-[2.4-dimethyl-thienyl-(3)]-1.3.4-thiadiazol
2-Amino-5-[4.5-dimethyl-thienyl-(3)]-1.3.4-thiadiazol
2-Amino-5-[4.5-dimethyl-furyl-(3)]-1.3.4-thiadiazol
2-Amino-5-[2.5-dimethyl-furyl-(3)]-1.3.4-thiadiazol
2-Amino-5-[3-methyl-pyridyl-(2)]-1.3.4-thiadiazol
2-Amino-5-[4-methyl-pyridyl-(2)]-1.3.4-thiadiazol
2-Amino-5-[5-methyl-pyridyl-(2)]-1.3.4-thiadiazol
2-Amino-5-[6-methyl-pyridyl-(2)]-1.3.4-thiadiazol
2-Amino-5-[2-methyl-pyridyl-(3)]-1.3.4-thiadiazol
2-Amino-5-[5-methyl-pyridyl-(3)]-1.3.4-thiadiazol
2-Amino-5-[6-methyl-pyridyl-(3)]-1.3.4-thiadiazol
2-Amino-5-[3,4-dimethyl-pyridyl-(2)]-1.3.4-thiadiazol
2-Amino-5-[2.4-dimethyl-pyridyl-(3)]-1.3.4-thiadiazol
2-Amino-5-(2-methyl-phenyl)-1.3.4-thiadiazol
2-Amino-5-(2-äthyl-phenyl)-1.3.4-thiadiazol
2-Amino-5-(2-isopropyl-phenyl)-1.3.4-thiadiazol
2-Amino-5-(3-methyl-phenyl)-1.3.4-thiadiazol
2-Amino-5-(3-butyl-phenyl)-1.3.4-thiadiazol
2-Amino-5-(4-methyl-phenyl)-1.3.4-thiadiazol
2-Amino-5-(2.3-dimethyl-phenyl)-1.3.4-thiadiazol
2-Amino-5-(2.4-dimethyl-phenyl)-1.3.4-thiadiazol
2-Amino-5-(2.6-dimethyl-phenyl)-1.3.4-thiadiazol
2-Amino-5-(2-methyl-5-äthylphenyl)-1.3.4-thiadiazol
2-Amino-5-(2-chlor-phenyl)-1.3.4-thiadiazol
2-Amino-5-(2-fluor-phenyl)-1.3.4-thiadiazol

2-Amino-5-(3-brom-phenyl)-1.3.4-thiadiazol
2-Amino-5-(4-chlor-phenyl)-1.3.4-thiadiazol
2-Amino-5-(4-phenyl-phenyl)-1.3.4-thiadiazol
2-Amino-5-(3-trifluor-methyl)-1.3.4-thiadiazol
2-Amino-5-(4-trifluor-methyl)-1.3.4-thiadiazol
2-Amino-5-(2-hydroxy-phenyl)-1.3.4-thiadiazol
2-Amino-5-(2-acetoxy-phenyl)-1.3.4-thiadiazol
2-Amino-5-(3-hydroxy-phenyl)-1.3.4-thiadiazol
2-Amino-5-(4-hydroxy-phenyl)-1.3.4-thiadiazol
2-Amino-5-(2-methoxy-phenyl)-1.3.4-thiadiazol
2-Amino-5-(3-methoxy-phenyl)-1.3.4-thiadiazol
2-Amino-5-(4-methoxy-phenyl)-1.3.4-thiadiazol
2-Amino-5-(2-äthoxy-phenyl)-1.3.4-thiadiazol
2-Amino-5-(3-isopropoxy-phenyl)-1.3.4-thiadiazol
2-Amino-5-(4-butoxy-phenyl)-1.3.4-thiadiazol
2-Amino-5-(2-methylmercapto-phenyl)-1.3.4-thiadiazol
2-Amino-5-(2-methylsulfonyl-phenyl)-1.3.4-thiadiazol
2-Amino-5-(3-methylmercapto-phenyl)-1.3.4-thiadiazol
2-Amino-5-(4-äthylmercapto-phenyl)-1.3.4-thiadiazol
2-Amino-5-(4-äthylsulfonyl-phenyl)-1.3.4-thiadiazol
2-Amino-5-(2-methylsulfinyl-phenyl)-1.3.4-thiadiazol
2-Amino-5-(2-nitro-phenyl)-1.3.4-thiadiazol
2-Amino-5-(3-nitro-phenyl)-1.3.4-thiadiazol
2-Amino-5-(2-cyano-phenyl)-1.3.4-thiadiazol
2-Amino-5-(3-cyano-phenyl)-1.3.4-thiadiazol
2-Amino-5-(4-cyano-phenyl)-1.3.4-thiadiazol
2-Amino-5-(2-methyl-4-chlor-phenyl)-1.3.4-thiadiazol
2-Amino-5-(2-methyl-5-chlor-phenyl)-1.3.4-thiadiazol
2-Amino-5-(2-methyl-4-fluor-phenyl)-1.3.4-thiadiazol
2-Amino-5-(2.4-dimethyl-phenyl)-1.3.4-thiadiazol
2-Amino-5-(3.4-dimethyl-phenyl)-1.3.4-thiadiazol
2-Amino-5-(2.6-dimethyl-phenyl)-1.3.4-thiadiazol
2-Amino-5-(2-chlor-6-methyl-phenyl)-1.3.4-thiadiazol
2-Amino-5-(2-fluor-4-methyl-phenyl)-1.3.4-thiadiazol
2-Amino-5-(2.6-dichlor-phenyl)-1.3.4-thiadiazol
2-Amino-5-(2.4-dichlor-phenyl)-1.3.4-thiadiazol
2-Amino-5-(3.5-dichlor-phenyl)-1.3.4-thiadiazol
2-Amino-5-(2-hydroxy-4-methyl-phenyl)-1.3.4-thiadiazol
2-Amino-5-(2-hydroxy-4-chlor-phenyl)-1.3.4-thiadiazol
2-Amino-5-(2-acetoxy-4-chlor-phenyl)-1.3.4-thiadiazol
2-Amino-5-(3-acetoxy-4-methyl-phenyl)-1.3.4-thiadiazol
2-Amino-5-(3-methylmercapto-4-chlor-phenyl)-1.3.4- thiadiazol
2-Amino-5-(3.4-dimethoxy-phenyl)-1.3.4-thiadiazol
2-Amino-5-(2.4-dimethoxy-phenyl)-1.3.4-thiadiazol
2-Amino-5-(2-methoxy-4-chlor-phenyl)-1.3.4-thiadiazol
2-Amino-5-(2-methyl-4-methoxy-phenyl)-1.3.4-thiadiazol
2-Amino-5-(2-nitro-4-chlor-phenyl)-1.3.4-thiadiazol
2-Amino-5-(2-methyl-4-nitro-phenyl)-1.3.4-thiadiazol
2-Amino-5-(2-methyl-benzyl)-1.3.4-thiadiazol
2-Amino-5-(2-chlor-benzyl)-1.3.4-thiadiazol
2-Amino-5-(α-methyl-2.6-dichlorbenzyl)-1.3.4-thiadiazol
2-Amino-5-(3.4-dichlor-benzyl)-1.3.4-thiadiazol
2-Amino-5-(4-chlor-benzyl)-1.3.4-thiadiazol
2-Amino-5-(2-methoxy- benzyl)-1.3.4-thiadiazol
2-Amino-5-(4-nitro-benzyl)-1.3.4-thiadiazol
2-Amino-5-(2-methyl-4-chlor-benzyl)-1.3.4-thiadiazol
2-Amino-5-(α-methyl-3.4-dimethyl-benzyl)-1.3.4-thiadiazol

Die als Ausgangsmaterial verwendeten Carbonylverbindungen der allgemeinen Formel II sind bekannt oder können nach literaturbekannten Methoden erhalten werden (vergl. z.B. Houben-Weyl, Methoden der organischen Chemie Band V, 3 S. 615–622; Band V, 4. S. 171–189, Georg Thieme Verlag, Stuttgart 1962 bzw. 1960.

Als Beispiele seien genannt:

α-Chloracetophenon
α-Brom-acetophenon
Phenacyl-N-pyridinium-bromid
α-Brom-2-chlor-acetophenon
α-Brom-3-chlor-acetophenon
α-Brom-4-chlor-acetophenon
α-Brom-3.4-dichlor-acetophenon
α-Brom-4-trifluormethyl-acetophenon
α-Brom-2-methylmercapto-acetophenon
α-Brom-2-methyl-acetophenon
α-Brom-3-methyl-acetophenon
α-Brom-4-methyl-acetophenon
α-Brom-2-methyl-4-chlor-acetophenon
α-Brom-4-methoxy-acetophenon
α-Brom-3-nitro-acetophenon
α-Brom-4-nitro-acetophenon
α-Brom-4-cyano-acetophenon
α-Brom-4-methylmercapto-acetophenon
α-Brom-4-methylsulfonyl-acetophenon
α-Brom-4-tert.-butyl-acetophenon
α-Brom-4-phenyl-acetophenon
α-Brom-4-brom-acetophenon
α-Brom-4-fluor-acetophenon
α-Brom-2-fluor-acetophenon
α-Brom-propiophenon
α-Brom-4-methyl-propiophenon
α-Brom-3-chlor-propiophenon
α-Brom-3-methyl-propiophenon
α-Brom-α-phenyl-acetophenon (Desylbromid)
α-Brom--α-phenyl-4-chlor-acetophenon

Die als Ausgangsstoffe verwendeten 1-Acylamino-2-mercaptoimidazole können nach literaturbekannten Verfahren (vergl. T. Pyl et al, Lieb. Ann. d. Chemie 663 [1963], S. 113–119) erhalten werden.

Als Beispiele seien u.a. genannt:

1-(2-Chlorbenzoylamino)-2-mercapto-4-phenylimidazol

1-(4-Chlorbenzoylamino)-2-mercapto-4-phenyl-imidazol
1-(2-Methylbenzoylamino)-2-mercapto-4-phenylimidazol
1-(3-Methylbenzoylamino)-2-mercapto-4-phenylimidazol
1-(2-Methoxybenzoylamino)-2-mercapto-4-phenylimidazol
1-(2-Hydroxybenzoylamino)-2-mercapto-4-phenylimidazol
1-(3-Nitrobenzoylamino)-2-mercapto-4-phenyl-imidazol
1-(2-Trifluormethylbenzoylamino)-2-mercapto-4-phenyl-imidazol
1-(α-Furoylamino)-2-mercapto-4-phenyl-imidazol
1-(α-Thenoylamino)-2-mercapto-4-phenyl-imidazol

Bei der Durchführung der erfindungsgemässen Verfahren können als Verdünnungsmittel alle üblichen inerten organischen Lösungsmittel eingesetzt werden. Hierzu gehören vorzugsweise Kohlenwasserstoffe wie Benzol, Toluol, Xylol, Halogenkohlenwasserstoffe wie Methylenchlorid, Chloroform, Tetrachlorkohlenstoff, Chlorbenzol, Alkohole wie Methanol, Äthanol, Propanol, Butanol, Benzylalkohol, Glykolmonomethyläther, Äther wie Tetrahydrofuran, Dioxan, Glykoldimethyläther, Amide wie Dimethylformamid, Dimethylacetamid, N-Methyl-pyrrolidon, Hexamethylphosphorsäuretriamid, Sulfoxyde wie Dimethylsulfoxyd, Sulfone wie Sulfolan und Basen wie Pyridin, Pikolin, Collidin, Lutidin und Chinolin.

Die Reaktionstemperaturen können in einem grossen Bereich variiert werden. Im allgemeinen arbeitet man zwischen 30 und 230°C, vorzugsweise zwischen 50 und 180°C. Man arbeitet bei Normaldruck, es kann jedoch auch in geschlossenen Gefässen bei höherem Druck gearbeitet werden.

Bei der Durchführung des erfindungsgemässen Verfahrens (Variante A) werden die Verbindungen der Formel (II) und (III) jeweils in molaren Mengen miteinander umgesetzt.

Die erfindungsgemäss erhaltenen Verbindungen sind als Arzneimittel verwendbare Substanzen. Sie bewirken bei oraler oder parenteraler Anwendung eine starke Verminderung der thrombotischen Abscheidung und können daher zur Behandlung und Prophylaxe thromembolischer Erkrankungen eingesetzt werden.

Die neuen Wirkstoffe können in bekannter Weise in die üblichen Formulierungen überführt werden wie Tabletten, Kapseln, Dragees, Emulsionen, Suspensionen und Lösungen, unter Verwendung inerter nichttoxischer, pharmazeutisch geeigneter Trägersubstanzen oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden.

Als Hilfsstoffe seien beispielhaft aufgeführt: Wasser, nichttoxische organische Lösungsmittel wie Paraffine (z.B. Erdölfraktionen), pflanzliche Öle (z.B. Erdnuss-/Sesamöl), Alkohole (z.B. Äthylalkohol, Glycerin), Glykole (z.B. Propylenglykol, Polyäthylenglykol), feste Trägerstoffe wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel wie nichtionogene und anionische Emulgatoren (z.B. Polyäthylen-Fettsäure-Ester, Polyoxyäthylen-Fettalkohol-Äther, Alkylsulfonate und Arylsulfonate), Dispergiermittel (z.B. Lignin, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral.

Im Falle der oralen Anwendung können Tabletten selbstverständlich ausser den genannten Trägerstoffen auch Zusätze wie Natriumcitrat, Calciumcarbonat und Calciumphosphat zusammen mit verschiedenen Zuschlagsstoffen wie Stärke, vorzugsweise Kartoffelstärke, Gelatine und dergleichen enthalten. Weiterhin können Gleitmittel wie Magnesiumstearat, Natriumlaurylsulfat und Talkum zum Tablettieren mitverwendet werden. Im Falle wässriger Suspensionen und/oder Elixieren, die für orale Anwendungen gedacht sind, können die Wirkstoffe ausser mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden. Als besonders vorteilhaft für den Fall der parenteralen Anwendung hat sich die Tatsache herausgestellt, dass die erfindungsgemässen Verbindungen in einem geeigneten Lösungsmittel mit der äquimolaren Menge einer nichttoxischen anorganischen oder organischen Base vereinigt. Als Beispiele seien genannt: Natronlauge, Kalilauge, Äthanolamin, Diäthanolamin, Triäthanolamin, Amino-tris-hydroxymethyl-methan, Glucosamin, N-Methylglucosamin.

Derartige Salze können auch für die orale Anwendung der erfindungsgemässen Verbindungen eine erhöhte Bedeutung besitzen, indem sie die Resorption je nach Wunsch beschleunigen oder verzögern. Als Beispiele seien ausser den oben bereits erwähnten Salzen genannt: Magnesiumsalz, Calciumsalze, Aluminiumsalz und Eisensalze.

Im allgemeinen hat es sich als vorteilhaft erwiesen, bei parenteraler Applikation Mengen von etwa 0,01 bis 50 mg/kg, vorzugsweise 0,1 bis 10 mg/kg Körpergewicht pro Tag zur Erzielung wirksamer Ergebnisse zu verabreichen, und bei oraler Applikation beträgt die Dosierung etwa 0,1 bis 50 mg/kg, vorzugsweise 0,5 mg bis 100 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in wenigen Fällen ausreichend sein, mit weniger als der vorgenannten Mindestmenge auszukommen, während in anderen Fällen die genannte obere Grenze überschritten werden muss. Im Fall der Applikation grösserer Mengen kann es empfehlenswert sein, diese in mehrere Einzelgaben über den Tag zu verteilen.

Diese Angaben gelten sowohl für die Anwendung der erfindungsgemässen Verbindungen in der Veterinär- als auch in der Humanmedizin.

Die antithrombotische Wirkung der erfindungsgemässen Verbindungen wird an experimentell erzeugten venösen Thromben der Ratte getestet. Hierbei zeigt sich, dass peroral applizierte Dosen von 10 mg/kg bereits eine Hemmung des Trombuswachstums von mehr als 30% bewirken. Die gleiche Dosis ergab eine Hemmung des Thrombusgewichtes bei arteriellen Thromben der Ratte von über 60%.

Neben dieser antitrombotischen Wirkung zeigen die erfindungsgemässen Verbindungen auch eine thrombolytische Wirkung, d.h. sie reduzieren bei nachträglicher Applikation die Grösse bereits vorhandener Thromben bzw. lösen sie wieder auf. Entsprechende thrombolytische Effekte konnten bisher nur durch wiederholte intravenöse Applikation toxischer Fibrinolytika wie Streptokinase und Urokinase erzielt werden. Diese vorteilhafte Wirkung kann bereits durch täglich einmalige orale Applikation der erfindungsgemässen Verbindungen erreicht werden.

Beispiel 1
(Verfahrensvariante A)

a) 2-(α-Furyl)-6-phenyl-imidazo[2.1-b]-1,3,4-thiadiazol

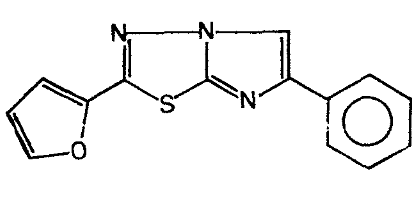

16,7 g (0,1 Mol) 2-Amino-5-(α-furyl)-1,3,4-thiadiazol (hergestellt durch Oxidation von Furfurolthiosemicarbazon) werden zusammen mit 20 g Bromacetophenon in 100 ml Dimethylformamid 5 Stunden auf 100°C unter Rühren erhitzt. Man kühlt, saugt ausgefallenes Reaktionsprodukt ab und engt die Mutterlauge im Rotaverdampfer ein. Der ölige Rückstand wird mit Wasser verrieben und nach dem Abdekantieren in Äthanol aufgekocht, wobei der Rückstand durchkristallisiert. Man saugt ab und vereinigt mit der Hauptmenge. Insgesamt werden 21,2 g (79,5%) farblose Kristalle von F 190°–192°C erhalten.

b) 8,4 g (0,05 Mol) 2-Amino-5-(α-furyl)-1,3,4-thiadiazol werden zusammen mit 10 g Bromacetophenon in 100 ml Aceton 2 Stunden unter Rühren rückfliessend zum Sieden erhitzt. Man kühlt, saugt ab und erhält 17 g eines farblosen Kristallisats vom F. 219°C als Hydrobromid. Die mit 2n NaOH in Freiheit gesetzte Base (2-(α-Furyl)-4-(2-phenacyl)-1,3,4-thiadiazolon-imid-(5) der Formel

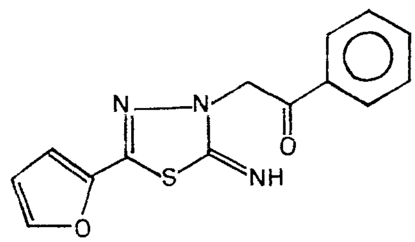

schmilzt (aus Äthanol) bei 166°C.

1. 3,7 g (0,01 Mol) des Hydrobromids vom F. 219°C werden in 80 ml Wasser 2 Stunden unter Rühren rückfliessend erhitzt. Man kühlt ab und saugt das ausgeschiedene Produkt ab. Es werden 2,2 g farblose Kristalle (82% d. Th.) vom F. 190°–192°C erhalten, die mit dem nach a) erhaltenen Produkt identisch sind.

2. 5 g des Hydrobromids vom F. 210°C werden in 25 ml Dimethylformamid 0,5 Stunden unter Rühren auf 150°C erwärmt. Man verjagt das Lösungsmittel im Rotaverdampfer, verreibt mit Wasser, dekantiert das Wasser ab und kocht mit 50 ml Äthanol auf, kühlt und saugt ab. Man erhält 3,5 g farblose Kristalle (96% d.Th.) vom F. 190°–192°C, die mit dem nach a) erhaltenen Produkt identisch sind.

Beispiel 2

2-(α-Furyl)-6-(4-chlorphenyl)-imidazo[2.1-b]-1,3,4-thiadiazol

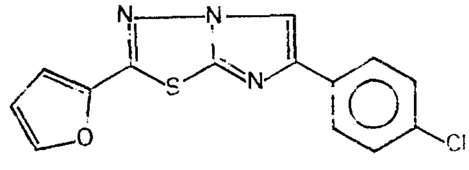

16,7 g (0,1 Mol) 2-Amino-5-(α-furyl)-1,3,4-thiadiazol werden zusammen mit 23,4 g 4-Chlor-α-bromacetophenon in 100 ml Dimethylformamid 5 Stunden unter Rühren zum Sieden erhitzt. Man kühlt, saugt ab und digeriert in heissem Methanol. Nach dem Abkühlen wird erneut abgesaugt und mit Methanol nachgewaschen. Man erhält 22,2 g farblose Kristalle, F. 204°C, Ausbeute 75% d. Th.

Beispiel 3

2-(α-Furyl)-6-(4-methoxyphenyl)-imidazo[2.1-b]-1,3,4-thiadiazol

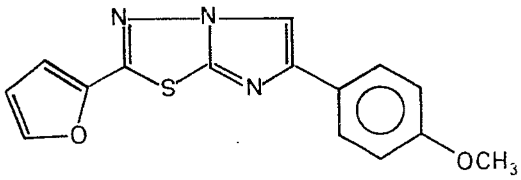

16,7 g (0,1 Mol) 2-Amino-5-(α-furyl)-1,3,4-thiadiazol werden zusammen mit 22,8 g 4-Methoxy-α-bromacetophenon in 200 ml Dimethylformamid 3 Stunden lang unter Rühren rückfliessend erhitzt. Man kühlt, saugt ab und digeriert das ausgefallene Produkt mit heissem Alkohol. Man erhält 66,5 g farblose Kristalle vom F. 197°C entsprechend 66,5% d. Th.

In analoger Arbeitsweise wurden die in Tabelle 1 aufgeführten Verbindungen erhalten.

Tabelle 1

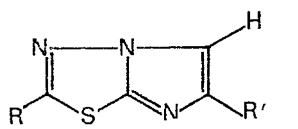

| Beispiel Nr. | R | R′ | F | Ausbeute % d. Th. |
|---|---|---|---|---|
| 4 | 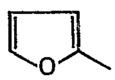 | 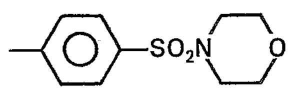 | 299°C | 80 |
| 5 | 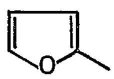 | 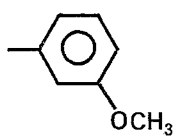 | 161°C | 51 |
| 6 | 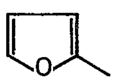 | 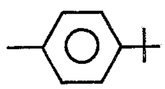 | 204° | 67 |
| 7 | 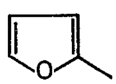 | 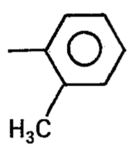 | 130° | 35 |
| 8 | 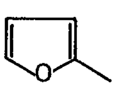 | 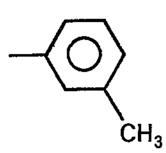 | 127° | 57 |
| 9 | 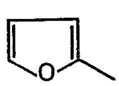 | 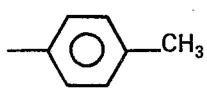 | 202° | 55 |
| 10 | 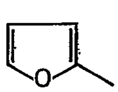 | 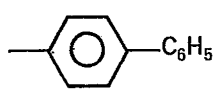 | 260° | 66 |
| 11 | 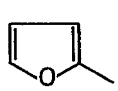 | 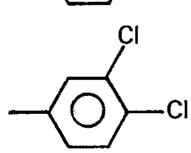 | 214° | 68 |

Tabelle 1 (Fortsetzung)

| Beispiel Nr. | R | R' | F | Ausbeute % d. Th. |
|---|---|---|---|---|
| 12 | 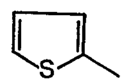 | 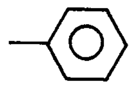 | 192° | 70 |
| 13 | 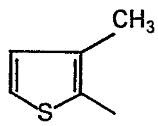 | 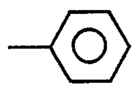 | 142° | 55 |
| 14 | 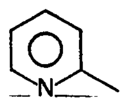 | 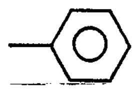 | 237° | 73 |
| 15 | 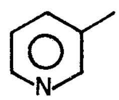 | 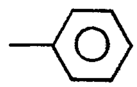 | 217° | 10 |

Beispiel 16
2-(2-Chlorphenyl)-6-phenyl-imidazo[2.1-b]-1,3,4-thiadiazol

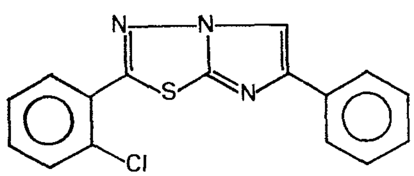

10,5 g (0,05 Mol) 2-Amino-5-(2-chlorphenyl)-1,3,4-thiadiazol, F. 189,5°C (dargestellt durch Umsetzung von Thiosemicarbazid mit 2-Chlorbenzoylchlorid) werden zusammen mit 10 g (0,05 Mol) Bromacetophenon in 100 ml DMF 3 Stunden auf 120°C erwärmt, dann gekühlt und am Rotaverdampfer eingeengt. Nach dem Digerieren mit Wasser wird abgesaugt und der Rückstand aus Alkohol umkristallisiert. Durch Einengen der alkoholischen Mutterlauge wird eine zweite Fraktion gewonnen. Gesamtausbeute 12,5 g farblose Kristalle vom F. 126°C entsprechend 80% d. Th.

In analoger Arbeitsweise wurden folgende 2-Aryl-6-phenyl-imidazo[2.1-b]-1,3,4-thiadiazole der Tabelle 2 erhalten:

Tabelle 2

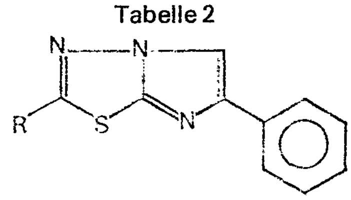

| Beispiel Nr. | R | F | Ausbeute % d. Th. |
|---|---|---|---|
| 17 | 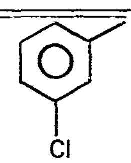 | 191° | 70 |
| 18 | 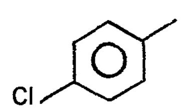 | 260° | 72 |
| 19 | 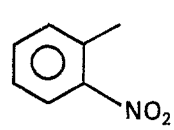 | 182°C | 45 |

Tabelle 2 (Fortsetzung)

| Beispiel Nr. | R | F | Ausbeute % d. Th. |
|---|---|---|---|
| 20 | 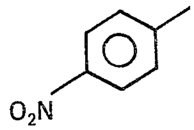 | 268° | 63 |
| 21 | 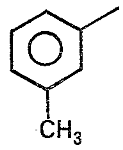 | 155° | 60 |
| 22 | 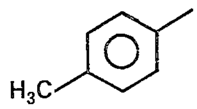 | 219° | 77 |
| 23 | 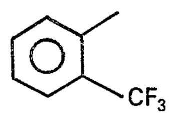 | 156° | 58 |
| 24 | 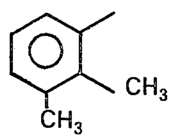 | 180° | 69 |
| 25 | 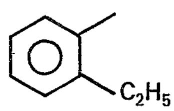 | 100° | 51 |
| 26 | 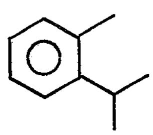 | 132° | 47 |
| 27 | 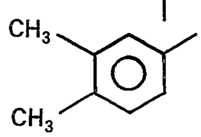 | 183° | 67 |
| 28 | 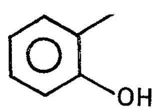 | 284° | 64 |
| 29 | 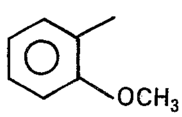 | 156° | 36 |
| 30 | 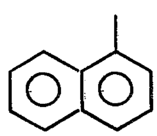 | 192° | 55 |
| 31 | 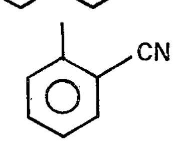 | 217° | 40 |

Weiterhin wurden in analoger Weise folgende 2-Aralkyl-6-aryl-imidazo[2.1-b]-1,3,4-thiadiazole der Tabelle 3 dargestellt:

Tabelle 3

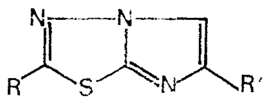

| Beispiel Nr. | R | R′ | F | Ausbeute % d. Th. |
|---|---|---|---|---|
| 32 | 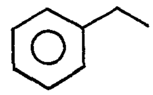 | 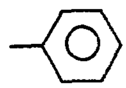 | 150°C | 63 |
| 33 | 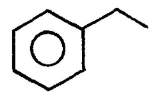 | 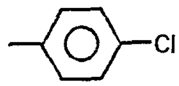 | 163° | 77 |
| 34 | 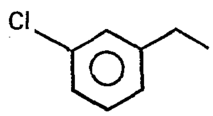 | 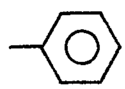 | 117° | 67 |
| 35 | 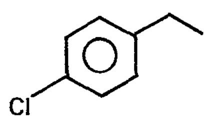 | 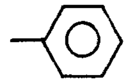 | 205° | 57 |
| 36 | 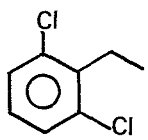 | 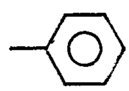 | 170° | 80 |
| 37 | 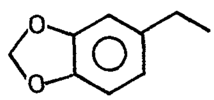 | 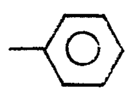 | 169° | 51 |
| 38 | 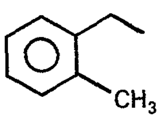 | 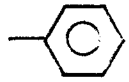 | 176° | 78 |
| 39 | 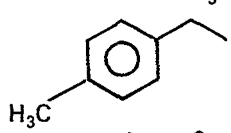 | 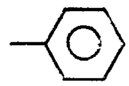 | 201° | 76 |
| 40 | 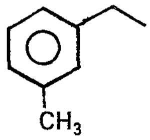 | 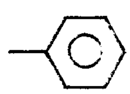 | 129° | 53 |
| 41 | 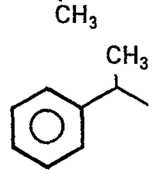 | 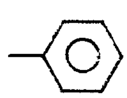 | 125° | 63 |

Beispiel 42

2-(2-Methylphenyl)-6-phenyl-imidazo[2.1-b]-1,3,4-thiadiazol

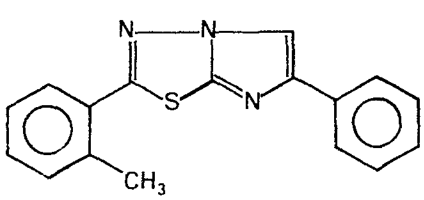

(Verfahrensvariante A)

a) 19,1 g (0,1 Mol) 2-Amino-5-(2-methylphenyl)-1,3,4-thiadiazol, F. 193°C, werden zusammen mit 20 g α-Bromacetophenon in 100 ml Dimethylformamid 3 Stunden auf 120°C unter Rühren erhitzt. Man destilliert das Lösungsmittel weitgehend ab, verreibt mit Wasser, saugt ab und kristallisiert aus Alkohol um. Man erhält 18,6 g farblose Kristalle, F. 160°C, entsprechend 65% d. Th.

b) 19,1 g (0,1 Mol) 2-Amino-5-(2-methylphenyl)-1,3,4-thiadiazol werden zusammen mit 20 g Bromacetophenon in 200 ml Aceton 7 Stunden unter Rühren gekocht. Man saugt in der Kälte ab und erhitzt das Kristallisat über Nacht unter Rühren in siedendem Wasser. Man stellt neutral, saugt ab und kristallisiert aus Alkohol um, F. 160°C, Ausbeute 21 g entsprechend 72% d. Th.

(Verfahrensvariante B)

4,4 g 1-(2-Methylbenzoylamino)-2-mercapto-4-phenylimidazol (erhalten durch Hydrazinolyse von 2-Benzylmercapto-6-phenyl-imidazo[2.1-b]-1,3,4-thiadiazol und anschliessende Acylierung des 1-Amino-2-mercapto-4-phenylimidazols mit 2-Methylbenzoylchlorid), F. 268–270°C, wird in 30 ml $POCl_3$ 20 Minuten zum Sieden erhitzt. Man kühlt ab, zersetzt mit Eis, saugt ab und kristallisiert anschliessend aus Alkohol um. Man erhält 2,6 g farblose Kristalle vom F. 159–160°C, entsprechend 67% d. Th.

Nach Verfahrensvariante A wurden in analoger Arbeitsweise die in Tabelle 4 genannten 2-(2-Methylphenyl)-6-aryl-imidazo[2.1-b]-1.3.4-thiadazole erhalten:

Tabelle 4

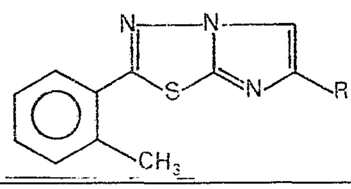

| Beispiel Nr. | R | F | Ausbeute % d. Th. |
|---|---|---|---|
| 43 | 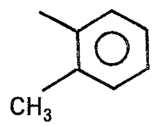 | 115°C | 27 |
| 44 | 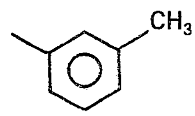 | 137°C | 67 |
| 45 | 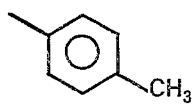 | 203°C | 60 |
| 46 | 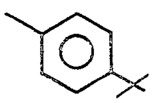 | 127°C | 65 |
| 47 | 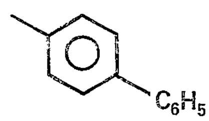 | 208°C | 60 |
| 48 | 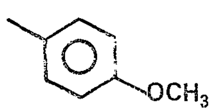 | 159°C | 42 |
| 49 | 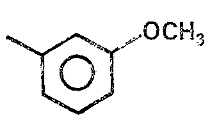 | 172°C | 64 |
| 50 | 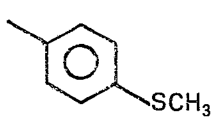 | 170°C | 52 |
| 51 | 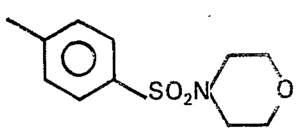 | 217° | 25 |

Tabelle 4 (Fortsetzung)

| Beispiel Nr. | R | F | Ausbeute % d. Th. |
|---|---|---|---|
| 52 | 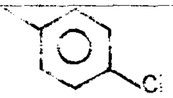 | 186° | 75 |
| 53 | 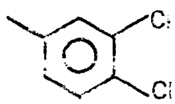 | 160° | 64 |

Die nachfolgenden Ausführungsbeispiele illustrieren die an sich bekannten Nachfolgereaktionen, in denen der Substituent $R^5$ alkyliert, acyliert, hydrolysiert oder verestert werden kann.

Beispiel 54

2-(2-Carboxyphenyl)-6-phenyl-imidazo[2.1-b]-1.3.4-thiadiazol

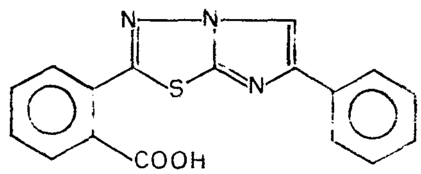

12 g 2-(2-Cyanophenyl)-6-phenyl-imidazo[2.1-b]-1.3.4-thiadiazol (Beispiel Nr. 31) werden in 40 g kristalliner $H_3PO_4$ 5 Stunden auf 170–175°C erhitzt, man kühlt, verrührt mit Wasser, saugt ab, löst in 2n NaOH, klärt mit Kohle und fällt mit 2n HCl aus.

Die Ausbeute beträgt 9,2 g farblose Kristalle entsprechend 72% d. Th. vom F. 251°C.

IR-Spektrum: Carboxyl-Carbonyl bei 1703 $cm^{-1}$; $M^+$ 321

Beispiel 55

2-(2-Äthoxycarbonylphenyl)-6-phenyl-imidazo[2.1-b]-1.3.4-thiadiazol

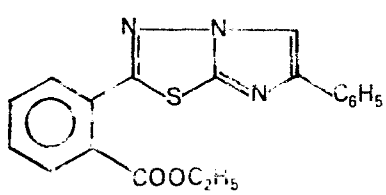

5 g der im vorstehenden Beispiel beschriebenen Verbindung werden in 25 ml Thionylchlorid 2 Stunden rückfliessend erhitzt. Man verjagt das Thionylchlorid, wäscht den Rückstand mit absolutem Äther, dekantiert den Äther ab und löst in absolutem Äthanol auf. Man lässt 1 Stunde stehen, dampft den überschüssigen Alkohol ab und kristallisiert den amorphen Rückstand aus 70%igem Äthanol um.

Man erhält 2 g farblose Kristalle vom F. 112–114°C. IR-Spektrum: Estercarbonyl 1711 $cm^{-1}$.

Beispiel 56

2-(Oxymethylencarbäthoxyphenyl)-6-phenyl-imidazo[2.1-b]-1.3.4-thiadiazol

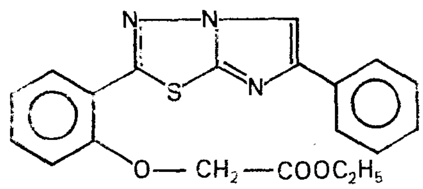

Man löst 1,2 g Natrium in 150 ml absol. Äthanol. In diese Lösung werden 14,6 g 2-(2-Hydroxyphenyl)-6-phenyl-imidazo[2.1-b]-1.3.4-thiadiazol (Beispiel Nr. 28) gelöst und dann unter Rühren 9 g Bromessigsäureäthylester eingetropft, wobei 2 Stunden rückfliessend erhitzt wird. Man kühlt, saugt ab und wäscht das Kristallisat mit Wasser. Man erhält 9,5 g farblose, in Alkali unlösliche Kristalle vom F. 120°C. IR-Spektrum: Estercarbonyl 1725 $cm^{-1}$.

Beispiel 57

Bei der analogen Umsetzung von 2-(2-Hydroxyphenyl)-6-phenyl-imidazo[2.1-b]-1.3.4-thiadiazol mit α-Brompropionsäureäthylester wird die Verbindung der Formel

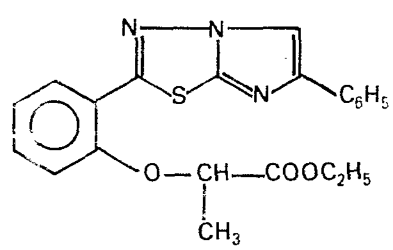

erhalten. F. 141°C, IR-Spektrum: Estercarbony 1709 $cm^{-1}$.

Beispiel 58

2-(2-Acetoxyphenyl)-6-phenyl-imidazo[2.1-b]-1.3.4-thiadiazol

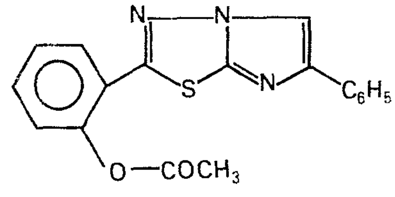

4,6 g 2-(2-Hydroxyphenyl)-6-phenyl-imidazo[2.1-b]-1.3.4-thiadiazol (Beispiel Nr. 28) werden in 75 ml Acetanhydrid ½ Stunde auf 150°C erwärmt, man dampft überschüssiges Acetanhydrid ab, verrührt mit Wasser und kristallisiert aus Äthanol um. Es werden 3,8 g farblose Kristalle vom F. 177°C erhalten entsprechend 72% d. Th.

IR-Spektrum: keine Hydroxylgruppe, Estercarbonyl bei 1744 $cm^{-1}$.

Beispiel 59

2-Furyl-5-methyl-6-phenyl-imidazo[2.1-b][1.3.4]-thiadiazol

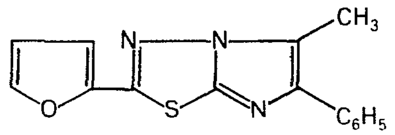

8,4 g 2-Amino-5-furyl-1.3.4-thiadiazol werden zusammen mit 12 g 2-Brompropiophenon in 50 ml Dimethylformamid 4 Stdn. auf 120°C unter Rühren erwärmt. Man kühlt ab, versetzt mit Wasser und kristallisiert den Niederschlag aus Isopropanol um. Es werden 5 g farblose Kristalle vom Fp.: 168°C erhalten.

Beispiel 60

2-(2-Methylphenyl)-5-methyl-6-phenyl-imidazo[2.1-b][1.3.4]-thiadiazol

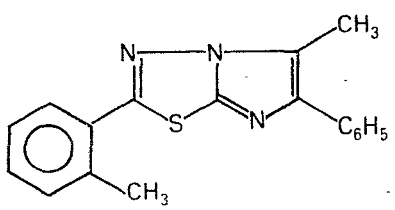

19,1 g 2-Amino-5-(2-Methylphenyl)-1.3.4-thiadiazol werden zusammen mit 23,5 g 2-Brompropiophenon in 200 ml Dimethylformamid 4 Stdn. auf 120°C erhitzt.

Man dampft ein, versetzt mit Wasser und kristallisiert aus Äthanol um.

Es werden 15 g farblose Kristalle vom Fp.: 144°C erhalten.

**Patentansprüche**

1. Imidazo-[2.1-b]-[1.3.4]-thiadiazole der allgemeinen Formel I

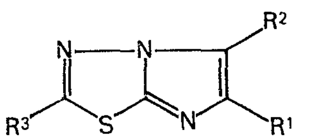

(I)

in welcher

$R^1$ für einen Phenylrest steht, der gegebenenfalls substituiert ist durch ein oder zwei gleiche oder verschiedene Substituenten aus der Gruppe Halogen, Trifluormethyl, Alkyl mit 1–6 C-Atomen, Alkoxy oder $SO_n$-Alkyl (n = 0 oder 2) mit jeweils 1–4 C-Atomen, Phenyl, Cyano, N-Methylsulfonamido, N-Dimethylsulfonamido oder Sulfonamido, deren N-Atom Glied eines 5–7gliedrigen Heterocyclus wie Pyrolidino, Piperidino oder Hexamethylenimino ist und der gegebenenfalls ein weiteres Sauerstoffatom enthalten kann,

$R^2$ für Wasserstoff, einen Alkylrest mit 1–3 C-Atomen oder einen Phenylrest, der unsubstituiert oder durch Fluor, Chlor, Methyl, Äthyl, Methoxy oder Äthoxy substituiert sein kann, steht,

und

$R^3$ für α- oder β-Naphthyl, einen α- oder β-Furylrest, der durch ein oder zwei Methyl- oder Äthylreste substituiert sein kann, einen α- oder β-Thienylrest, der durch ein oder zwei Methyl- oder Äthylreste substituiert sein kann, einen α- oder β-Pyridylrest, der durch Methyl oder Äthyl substituiert sein kann, einen Methylendioxybenzylrest oder einen Substituenten der Formel

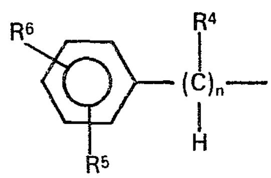

steht,

worin

n 0 oder 1 bedeutet,

$R^4$ Wasserstoff oder eine Methylgruppe bedeutet,

$R^5$ einen Substituenten aus der Gruppe Fluor, Chlor, Brom, Alkyl mit 1–4 C-Atomen oder Allyl, Phenyl, Trifluormethyl, Hydroxy, Alkoxy mit 1–4 C-Atomen, $SO_n$-Alkyl (n = 0–2) mit 1–2 C-Atomen im Alkylrest, Acyloxy mit bis zu 4 C-Atomen, Alkoxylencarboxy mit bis zu 6 C-Atomen, Oxyalkylencarbalkoxy mit bis zu 10 C-Atomen, Nitro, Cyano, Carboxy, Carbalkoxy mit bis zu 4 C-Atomen, bedeutet, oder für den Fall, dass n = 1 ist, Wasserstoff bedeutet,

$R^6$ Wasserstoff oder einen weiteren Substituenten aus der Gruppe Fluor, Chlor, Brom, Alkyl mit 1–4 C-Atomen oder einen Methoxy- oder Äthoxyrest bedeutet.

2. Verbindungen der Formel I gemäss Anspruch 1, in welcher

$R^1$ für Phenyl steht, das gegebenenfalls durch Fluor, Chlor, Methyl, tert.-Butyl, Phenyl, Trifluormethyl, Methoxy, Methylmercapto, Methylsulfonyl oder die

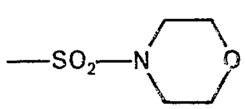 Gruppe substituiert ist,

$R^2$ für Wasserstoff, einen Methyl- oder Phenylrest steht,

$R^3$ α- oder β-Naphthyl, α- oder β-Furyl oder Thienyl bzw. α- oder β-Pyridyl bedeutet, wobei die heterocyclischen Reste durch eine Methylgruppe substituiert sein können, einen Methylendioxybenzylrest oder für einen Rest der Formel

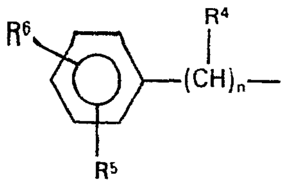

steht,

worin
n 0 oder 1 bedeutet und
$R^4$ Wasserstoff oder eine Methylgruppe bedeutet und
$R^5$ Methyl, Äthyl, Propyl, Isopropyl, Trifluormethyl, Fluor, Chlor oder Brom, Hydroxy, Methoxy, Äthoxy oder Propoxy, Oxymethylencarboxy, Oxymethylencarbomethoxy, Oxymethylencarbäthoxy, α-Oxyäthylencarboxy, α-Oxyäthylencarbomethoxy, α-Oxyäthylencarboäthoxy, Methylmercapto, Methylsulfonyl, Acetoxy, Propionyloxy, Nitro, Cyano, Carboxy, Carbomethoxy oder Carboäthoxy bedeutet, für den Fall, dass n = 1 ist, Wasserstoff bedeutet, und
$R^6$ Wasserstoff oder einen weiteren Substituenten aus der Gruppe Fluor, Chlor, Brom, Methoxy oder Äthoxy bedeutet.

3. Verbindungen gemäss Anspruch 1 zur Verwendung bei der Bekämpfung von Erkrankungen.

4. Verbindungen gemäss Anspruch 1 zur Verwendung bei der Bekämpfung von thrombembolischen Erkrankungen.

5. Arzneimittel enthaltend mindestens eine Verbindung gemäss Anspruch 1.

6. Verfahren zur Herstellung von Verbindungen der allgemeinen Formel I

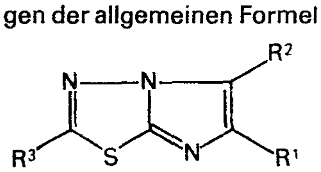

(I)

in welcher
$R^1$ für einen Phenylrest steht, der gegebenenfalls substituiert ist durch ein oder zwei gleiche oder verschiedene Substituenten aus der Gruppe Halogen, Trifluormethyl, Alkyl mit 1-6 C-Atomen, Alkoxy oder $SO_n$-Alkyl (n = 0 oder 2) mit jeweils 1-4 C-Atomen, Phenyl, Cyano,
N-Methylsulfonamido, N-Dimethylsulfonamido oder Sulfonamido, deren N-Atom Glied eines 5-7gliedrigen Heterocyclus wie Pyrolidino, Piperidino oder Hexamethylenimino ist und der gegebenenfalls ein weiteres Sauerstoffatom enthalten kann,
$R^2$ für Wasserstoff, einen Alkylrest mit 1-3 C-Atomen oder einen Phenylrest, der unsubstituiert oder durch Fluor, Chlor, Methyl, Äthyl, Methoxy oder Äthoxy substituiert sein kann, steht,
und
$R^3$ für α- oder β-Naphthyl, einen α- oder β-Furylrest, der durch ein oder zwei Methyl- oder Äthylreste substituiert sein kann, einen α- oder β-Thienylrest, der durch ein oder zwei Methyl- oder Äthylreste substituiert sein kann, einen α- oder β-Pyridylrest, der durch Methyl oder Äthyl substituiert sein kann, einen Methylendioxybenzylrest oder einen Substituenten der Formel

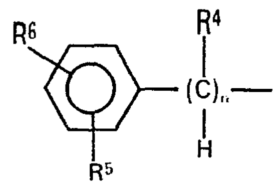

steht,

worin
n 0 oder 1 bedeutet,
$R^4$ Wasserstoff oder eine Methylgruppe bedeutet,
$R^5$ einen Substituenten aus der Gruppe
Fluor, Chlor, Brom, Alkyl mit 1-4 C-Atomen oder Allyl, Phenyl, Trifluormethyl, Hydroxy, Alkoxy mit 1-4 C-Atomen, $SO_n$-Alkyl (n = 0-2) mit 1-2 C-Atomen im Alkylrest, Acyloxy mit bis zu 4 C-Atomen, Alkoxylencarboxy mit bis zu 6 C-Atomen, Oxyalkylencarbalkoxy mit bis zu 10 C-Atomen, Nitro, Cyano, Carboxy, Carbalkoxy mit bis zu 4 C-Atomen bedeutet,
oder für den Fall, dass n = 1 ist, Wasserstoff bedeutet,
$R^6$ Wasserstoff oder einen weiteren Substituenten aus der Gruppe Fluor, Chlor, Brom, Alkyl mit 1 bis 4 C-Atomen oder einen Methoxy- oder Äthoxyrest bedeutet,
dadurch gekennzeichnet, dass man

A) Carbonylverbindungen der allgemeinen Formel II

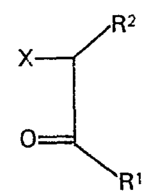

(II)

in welcher
$R^1$ und $R^2$ die oben angegebene Bedeutung haben und X für eine austretende Gruppe, insbesondere für Halogen, Hydroxy, Alkoxy, Acyloxy, Alkyl, Arylsulfonyloxy, quartäres Ammonium, tertiäres Phosphonium oder Sulfonium steht,
wobei die Carbonylverbindungen der Formel II auch in Form ihrer entsprechenden Enamine, Enolester oder Enoläther vorliegen können,
mit 2-Amino-1.3.4-thiadiazolen der allgemeinen Formel III

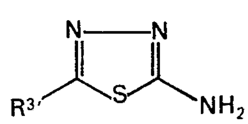

(III)

in welcher
$R^{3'}$ für Naphthyl, Furyl, Thienyl oder Pyridyl steht, die gegebenenfalls durch 1 oder 2 Methyl- oder Äthylreste substituiert sind, einen Methylendioxybenzylrest oder für einen Substituenten der allgemeinen Formel

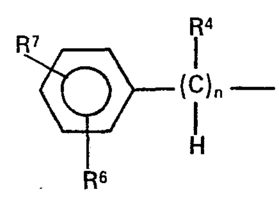

worin
n, $R^4$ und $R^6$ die oben angegebene Bedeutung haben und
$R^7$ für Fluor, Chlor, Brom, Alkyl, Allyl, Phenyl, Trifluormethyl, Hydroxy, Alkoxy, wobei die genannten Alkyl- und Alkoxyreste jeweils 1–4 C-Atome enthalten, $SO_n$-Alkyl (n = 0, 1 oder 2) mit 1 oder 2 C-Atomen im Alkylrest, Nitro oder Cyano steht, gegebenenfalls in Gegenwart inerter Lösungsmittel und basischer Hilfsstoffe bei Temperaturen zwischen 30 und 230°C umsetzt und gegebenenfalls in einer Nachfolgereaktion die Verbindungen der allgemeinen Formel I in welcher

a) $R^5$ für eine Hydroxylgruppe steht, diese Hydroxylgruppe in an sich bekannter Weise alkyliert oder acyliert wird oder

b) für den Fall, dass $R^5$ eine Nitrilgruppe bedeutet, diese Nitrilgruppe in an sich bekannter Weise zur Carboxygruppe verseift wird und gegebenenfalls anschliessend zu einer Carbalkoxygruppe verestert wird,
oder

B) wenn man 1-Acylamino-2-mercapto-imidazole der allgemeinen Formel IV

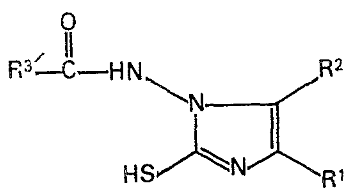

(IV)

in welcher
$R^1$, $R^2$ und $R^{3'}$ die oben angegebene Bedeutung haben,
mit wasserabspaltenden Mitteln, vorzugsweise mit anorganischen Säurehalogeniden, insbesondere mit Phosphoroxychlorid, bei Temperaturen zwischen 30 und 200°C, gegebenenfalls unter erhöhtem Druck, cyclisiert.

7. Verfahren zur Herstellung von Arzneimitteln, dadurch gekennzeichnet, dass man Verbindungen gemäss Anspruch 1 gegebenenfalls unter Zusatz von inerten pharmazeutisch unbedenklichen Hilfs- und Trägerstoffen in eine geeignete Applikationsform überführt.

**Claims**

1. Imidazo-[2,1-b]-[1,3,4]-thiadiazoles of the general formula I

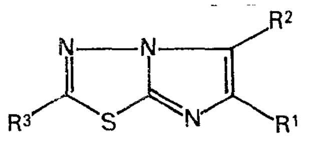

(I)

in which
$R^1$ represents a phenyl radical which is optionally substituted by one or two identical or different substituents from the group comprising halogen, trifluoromethyl, alkyl with 1 to 6 C-atoms, alkoxy or $SO_n$-alkyl (n = 0 or 2) with in each case 1 to 4 C-atoms, phenyl, cyano, N-methylsulphonamido, N-dimethylsulphonamido or sulphonamido, the N-atom of which is a member of a 5 to 7 membered heterocyclic ring, such as pyrrolidino, piperidino or hexamethyleneimino and which optionally can contain a further oxygen atom,
$R^2$ represents hydrogen, an alkyl radical with 1 to 3 C-atoms or a phenyl radical, which can be unsubstituted or substituted by fluorine, chlorine, methyl, ethyl, methoxy or ethoxy,
and
$R^3$ represents α- or β-naphthyl, an α- or β-furyl radical, which can be substituted by one or two methyl or ethyl radicals, an α- or β-thienyl radical, which can be substituted by one or two methyl or ethyl radicals, an α- or β-pyridyl radical, which can be substituted by methyl or ethyl, a methylenedioxybenzyl radical or a substituent of the formula

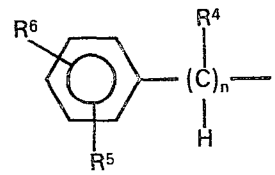

in which
$n_4$ denotes 0 or 1,
$R^4$ denotes hydrogen or an methyl group,
$R^5$ denotes a substituent from the group comprising fluorine, chlorine, bromine, alkyl with 1 to 4 C-atoms or allyl, phenyl, trifluoromethyl, hydroxyl, alkoxy with 1 to 4 C-atoms, $SO_n$-alkyl (n = 0–2) with 1 to 2 C-atoms in the alkyl radical, acyloxy with up to 4 C-atoms, alkoxylenecarboxyl with up to 6 C-atoms, oxyalkylenecarbalkoxy with up to 10 C-atoms, nitro, cyano, carboxyl, carbalkoxy with up to 4 C-atoms, or denotes hydrogen if n = 1,
$R^6$ denotes hydrogen or a further substituent from the group comprising fluorine, chlorine, bromine, alkyl with 1 to 4 C-atoms or a methoxy or ethoxy radical.

2. Compounds of the formula I according to Claim 1, in which
$R^1$ represents phenyl, which is optionally substituted by fluorine, chlorine, methyl, tert.-butyl, phenyl, trifluoromethyl, methoxy, methylmercapto, methylsulphonyl

or the 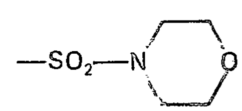 group,

$R^2$ represents hydrogen or a methyl or phenyl radical, $R^3$ denotes α- or β-naphthyl, α- or β-furyl or thienyl or α- or β-pyridyl, it being possible for the heterocyclic radicals to be substituted by a methyl group, or a methylenedioxybenzyl radical or a radical of the formula

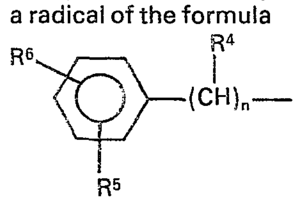

in which
n denotes 0 or 1 and
$R^4$ denotes hydrogen or a methyl group and
$R^5$ denotes methyl, ethyl, propyl, isopropyl, trifluoromethyl, fluorine, chlorine or bromine, hydroxyl, methoxy, ethoxy or propoxy, oxymethylenecarboxyl, oxymethylenecarboethoxy, α-oxyethylenecarboxyl, α-oxyethylenecarbomethoxy, α-oxyethylenecarboethoxy, methylmercapto, methylsulphonyl, acetoxy, propionyloxy, nitro, cyano, carboxyl, carbomethoxy or carboethoxy, or denotes hydrogen if n = 1, and
$R^6$ denotes hydrogen or a further substituent from the group comprising fluorine, chlorine, bromine, methoxy or ethoxy.

3. Compounds according to Claim 1 for use in combating diseases.

4. Compounds according to Claim 1 for use in combating thrombembolic diseases.

5. Medicaments containing at least one compound according to Claim 1.

6. Process for preparing compounds of the general formula I

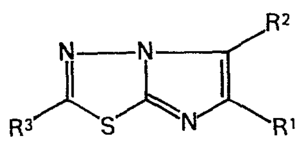

(I)

in which
$R^1$ represents a phenyl radical which is optionally substituted by one or two identical or different substituents from the group comprising halogen, trifluoromethyl, alkyl with 1 to 6 C-atoms, alkoxy or $SO_n$-alkyl (n = 0 or 2) with in each case 1 to 4 C-atoms, phenyl, cyano, N-methylsulphonamido, N-dimethylsulphonamido or sulphonamido, the N-atom of which is a member of a 5 to 7 membered heterocyclic ring, such as pyrrolidino, piperidino or hexamethyleneimino and which optionally can contain a further oxygen atom,
$R^2$ represents hydrogen, an alkyl radical with 1 to 3 C-atoms or a phenyl radical, which can be unsubstituted or substituted by fluorine, chlorine, methyl, ethyl, methoxy or ethoxy,
and
$R^3$ represents α- or β-naphthyl, an α- or β-furyl radical, which can be substituted by one or two methyl or ethyl radicals, an α- or β-thienyl radical, which can be substituted by one or two methyl or ethyl radicals, an α- or β-pyridyl radical, which can be substituted by methyl or ethyl, or a methylenedioxybenzyl radical or a substituent of the formula

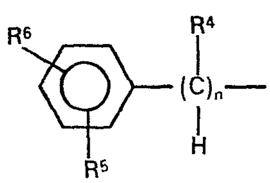

wherein
n denotes 0 or 1,
$R^4$ denotes hydrogen or a methyl group,
$R^5$ denotes a substituent from the group comprising fluorine, chlorine, bromine, alkyl with 1 to 4 C-atoms or allyl, phenyl, trifluoromethyl, hydroxyl, alkoxy with 1 to 4 C-atoms, $SO_n$-alkyl (n = 0–2) with 1–2 C-atoms in the alkyl radical, acyloxy with up to 4 C-atoms, alkoxylenecarboxyl with up to 6 C-atoms, oxyalkylenecarbalkoxy with up to 10 C-atoms, nitro, cyano, carboxyl, carbalkoxy with up to 4 C-atoms,
or denotes hydrogen if n = 1,
$R^6$ denotes hydrogen or a further substituent from the group comprising fluorine, chlorine, bromine, alkyl with 1 to 4 C-atoms or a methoxy or ethoxy radical, characterised in that

A) carbonyl compounds of the general formula II

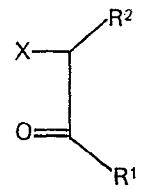

(II)

in which
$R^1$ and $R^2$ have the meaning indicated above and
X represents a leaving group, in particular halogen, hydroxyl, alkoxy, acyloxy, alkyl, arylsulphonyloxy, quaternary ammonium, tertiary phosphonium or sulphonium, it being possible for the carbonyl compounds of formula II also to be in the form of their corresponding enamines, enol esters or enol ethers,
are reacted with 2-amino-1,3,4-thiadiazoles of the general formula III

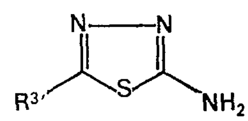

(III)

in which
$R^{3'}$ represents naphthyl, furyl, thienyl or pyridyl, which are optionally substituted by 1 or 2 methyl or ethyl radicals, a methylenedioxybenzyl radical or a substituent of the general formula

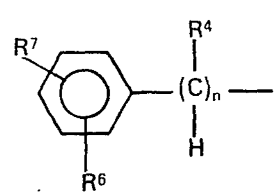

wherein
n, $R^4$ and $R^6$ have the meaning indicated above and $R^7$ represents fluorine, chlorine, bromine, alkyl, allyl, phenyl, trifluoromethyl, hydroxyl, alkoxy, the stated alkyl and alkoxy radicals in each case containing 1 to 4 C-atoms, $SO_n$-alkyl (n = 0, 1 or 2) with 1 or 2 C-atoms in the alkyl radical, nitro or cyano,

if appropriate in the presence of inert solvents and basic auxiliaries at temperatures between 30 and 230°C and if desired, in a subsequent reaction, the compounds of the general formula I in which

a) $R^5$ represents a hydroxyl group, this hydroxyl group is alkylated or acylated in a manner in itself known or

b) in the case where $R^5$ denotes a nitrile group, this nitrile group is saponified to the carboxyl group in a manner in itself known and the carboxyl group is then, if desired, esterified to a carbalkoxy group,

or

B) when 1-acylamino-2-mercapto-imidazoles of the general formula IV

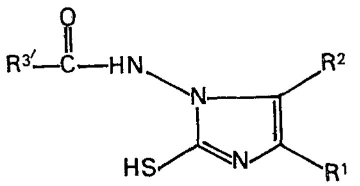

(IV)

in which

$R^1$, $R^2$ and $R^{3'}$ have the meaning indicated above, are cyclised with agents which split off water, preferably with inorganic acid halides, in particular with phosphorus oxychloride, at temperatures between 30 and 200°C and optionally under increased pressure.

7. Process for the preparation of medicaments, characterised in that compounds according to Claim 1 are converted into a suitable form of administration, if appropriate with the addition of inert pharmaceutically acceptable auxiliaries and excipients.

**Revendications**

1. Imidazo-[2.1-b]-[1.3.4]-thiadiazoles de formule générale I:

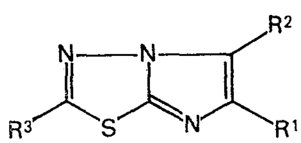

(I)

dans laquelle:

$R^1$ est un reste phényle qui est éventuellement substitué par un ou deux substituants égaux ou différents du groupe comprenant un halogène, un reste trifluorométhyle, un reste alkyle ayant 1 à 6 atomes de carbone, un reste alkoxy ou $SO_n$-alkyle (n = 0 ou 2) ayant chacun 1 à 4 atomes de carbones, le reste phényle, le reste cyano, le reste N-méthylsulfonamido, le reste N-diméthylsulfonamido ou un reste sulfonamido dont l'atome d'azote appartient à un hétérocycle pentagonal à heptagonal tel que pyrolidino, pipéridino ou hexaméthylène-imino et qui peut éventuellement renfermer un autre atome d'oxygène,

$R^2$ est l'hydrogène, un reste alkyle ayant 1 à 3 atomes de carbone ou un rest phényle qui peut ne pas être substitué ou être substitué par du fluor, du chlore, un groupe méthyle, éthyle, méthoxy ou éthoxy, et

$R^3$ est un reste α- ou β-naphtyle, un reste α- ou β-furyle qui peut être substitué par un ou deux restes méthyle ou éthyle, un reste α- ou β-thiényle qui peut être substitué par un ou deux restes méthyle ou éthyle, un reste α- ou β-pyridyle qui peut être substitué par un reste méthyle ou éthyle, un reste méthylènedioxybenzyle ou un substituant de formule:

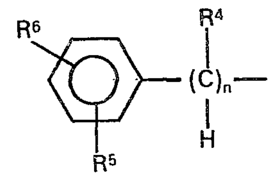

dans laquelle:

n est égal à 0 ou 1,

$R^4$ est l'hydrogène ou un groupe méthyle

$R^5$ est un substituant choisi dans le groupe comprenant le fluor, le chlore, le brome, un reste alkyle ayant 1 à 4 atomes de carbone ou un reste allyle, phényle, trifluorométhyle, hydroxy, alkoxy ayant 1 à 4 atomes de carbone, $SO_n$-alkyle (n = 0–2) ayant 1 ou 2 atomes de carbone dans le reste alkyle, un reste acyloxy ayant jusqu'à 4 atomes de carbone, alkoxylènecarboxy ayant jusqu'à 6 atomes de carbone, oxyalkylène-carbalkoxy ayant jusqu'à 10 atomes de carbone, nitro, cyano, carboxy, carbalkoxy ayant jusqu'à 4 atomes de carbone, ou bien représente l'hydrogène au cas où n est égal à 1,

$R^6$ est l'hydrogène ou un autre substituant choisi dans le groupe comprenant le fluor, le chlore, le brome, un reste alkyle ayant 1 à 4 atomes de carbone ou un reste méthoxy ou éthoxy,

2. Composés de formule I suivant la revendication 1, dans laquelle

$R^1$ est un reste phényle qui est éventuellement substitué par du fluor, du chlore, un groupe méthyle, tertio-butyle, phényle, trifluorométhyle, méthoxy, méthylmercapto, méthylsulfonyle ou le groupe:

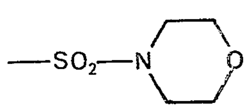

$R^2$ est l'hydrogène, un reste méthyle ou un reste phényle,

$R^3$ est un reste α- ou β-naphtyle, α- ou β-furyle ou thiényle ou un reste α- ou β-pyridyle, les restes hétérocycliques pouvant être substitués par un groupe méthyle, un reste méthylènedioxybenzyle ou un reste de formule:

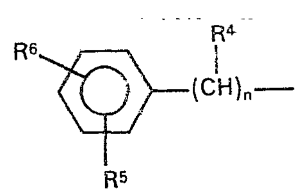

dans laquelle:
$n_4$ est égal à 0 ou 1 et
$R^4$ est l'hydrogène ou un groupe méthyle et
$R^5$ est le groupe méthyle, éthyle, propyle, isopropyle, trifluorométhyle, le fluor, le chlore ou le brome, le groupe hydroxy, méthoxy, éthoxy ou propoxy, oxyméthylène-carboxy, oxyméthylènecarbométhoxy, oxyméthylène-carbéthoxy, α-oxyéthylène-carboxy α-oxyéthylène-carbométhoxy, α-oxyéthylène-carbéthoxy, méthylmercapto, méthylsulfonyle, acétoxy, propionyloxy, nitro, cyano, carboxy, carbométhoxy ou carbéthoxy, représente l'hydrogène au cas où n est égal à 1, et
$R^6$ est l'hydrogène ou un autre substituant choisi dans le groupe comprenant le fluor, le chlore, le brome, le reste méthoxy ou le reste éthoxy.

3. Composés suivant la revendication 1, destinés à être utilisés dans la lutte contre des maladies

4. Composés suivant la revendication 1, destinés à être utilisés dans la lutte contre des maladies thromboemboliques.

5. Médicament contenant au moins un composé suivant la revendication 1.

6. Procédé de production de composés de formule générale I:

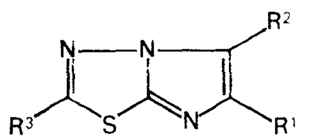
(I)

dans laquelle:
$R^1$ est un reste phényle qui est éventuellement substitué par un ou deux substituants égaux ou différents du groupe comprenant un halogène, un reste trifluorométhyle, un reste alkyle ayant 1 à 6 atomes de carbone, un reste alkoxy ou $SO_n$-alkyle (n = 0 ou 2) ayant chacun 1 à 4 atomes de carbone, le reste phényle, le reste cyano, le reste N-méthylsulfonamido, le reste N-diméthylsulfonamido ou un reste sulfonamido dont l'atome d'azote appartient à und hétérocycle pentagonal à heptagonal tel que pyrolidino, pipéridino ou hexaméthylène-imino et qui peut éventuellement renfermer un autre atome d'oxygène,
$R^2$ est l'hydrogène, un reste alkyle ayant 1 à 3 atomes de carbone ou un reste phényle qui peut ne pas être substitué ou être substitué par du fluor, du chlore, un groupe méthyle, éthyle, méthoxy ou éthoxy, et
$R^3$ est un reste α- ou β-naphtyle, un reste α- ou β-furyle qui peut être substitué par un ou deux restes méthyle ou éthyle, un reste α- ou β-thiényle qui peut être substitué par un ou deux restes méthyle ou éthyle, un reste α- ou β-pyridyle qui peut être substitué par un reste méthyle ou éthyle, un reste méthylènedioxybenzyle ou un substituant de formule:

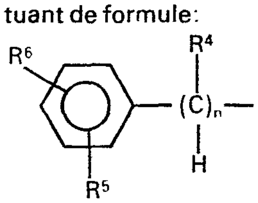

dans laquelle:
n est égal à 0 ou 1,
$R^4$ est l'hydrogène ou un groupe méthyle
$R^5$ est un substituant choisi dans le groupe comprenant le fluor, le chlore, le brome, un reste alkyle ayant 1 à 4 atomes de carbone ou un reste alkyle, phényle, trifluorométhyle, hydroxy, alkoxy ayant 1 à 4 atomes de carbone, $SO_n$-alkyle (n = 0–2) ayant 1 ou 2 atomes de carbone dans le reste alkyle, un reste acyloxy ayant jusqu'à 4 atomes de carbone, alkoxylènecarboxy ayant jusqu'à 6 atomes de carbone, oxyalkylène-carbalkoxy ayant jusqu'à 10 atomes de carbone, nitro, cyano, carboxy, carbalkoxy ayant jusqu'à 4 atomes de carbone, ou bien représente l'hydrogène au cas où n est égal à 1,
$R^6$ est l'hydrogène ou un autre substituant choisi dans le groupe comprenant le fluor, le chlore, le brome, un reste alkyle ayant 1 à 4 atomes de carbone ou un reste méthoxy ou éthoxy,
caractérisé en ce que:

A) on fait réagir des composés carbonyliques de formule générale II:

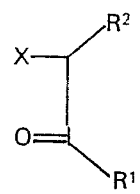
(II)

dans laquelle:
$R^1$ et $R^2$ ont la définition indiquée ci-dessus et
X est un groupe partant, en particulier un halogène, un groupe hydroxy, alkoxy, acyloxy, alkyle, arylsulfonyloxy, ammonium quaternaire, phosphonium ou sulfonium tertiaire,
les composés carbonyliques de formule II pouvant également se présenter sous la forme de leurs énamines, de leurs énolesters ou de leurs énoléthers correspondants,
avec des 2-amino-1,3,4-thiadiazoles de formule générale III:

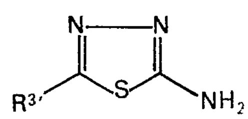
(III)

dans laquelle:
$R^{3'}$ représente les groupes naphtyle, furyle, thiényle ou pyridyle qui sont éventuellement substitués par un ou deux restes méthyle ou éthyle, un reste méthylène-dioxybenzyle ou un substituant de formule générale:

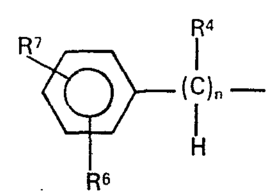

dans laquelle:
n, $R^4$ et $R^6$ ont la définition indiquée ci-dessus et
$R^7$ est du fluor, du chlore, du brome, un reste alkyle, allyle, phényle, trifluorométhyle, hydroxy, alkoxy, les restes alkyle et alkoxy mentionnés renfermant chacun 1 à 4 atomes de carbone, un reste $SO_n$-alkyle (n = 0, 1 ou 2) avec 1 ou 2 atomes de carbone dans le reste alkyle, le reste nitro ou le reste cyano,
éventuellement en présence de solvants inertes et de substances auxiliaires basiques, à des températures comprises entre 30 et 230°C et
le cas échéant, dans une réaction subséquente, les composés de formule générale I dans laquelle:

a) $R^5$ est un groupe hydroxyle, ce groupe hydroxyle est alkylé ou acylé d'un manière connue ou

b) pour le cas où $R^5$ représente un groupe nitrile, ce groupe nitrile est saponifié en groupe carboxy d'une manière connue et il est ensuite estérifié, le cas échéant, en un groupe carbalkoxy, ou

B) lorsqu'on cyclise des 1-acylamino-2-mercaptoimidazoles de formule générale IV:

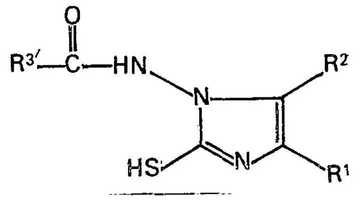

(IV)

dans laquelle:
$R^1$, $R^2$ et $R^3$ ont la définition indiquée ci-dessus, avec des agents éliminant de l'eau, de préférence avec des halogénures d'acides inorganiques, notamment avec l'oxychlorure de phosphore, à des températures comprises entre 30 et 200°C, éventuellement sous pression élevée.

7. Procédé de production de médicaments, caractérisé en ce qu'on fait prendre à des composés suivant la revendication 1 une forme propre à l'administration, éventuellement avec addition d'adjuvants et de supports inertes sans inconvénient du point de vue pharmaceutique.